(19) 

Europäisches
Patentamt

European
Patent Office

Office européen
des brevets

(11) **EP 4 706 501 A2**

(12) **EUROPEAN PATENT APPLICATION**

(43) Date of publication:
**11.03.2026  Bulletin 2026/11**

(21) Application number: **25200525.1**

(22) Date of filing: **05.09.2025**

(51) International Patent Classification (IPC):
**A61B 3/10** *(2006.01)*        **A61B 5/00** *(2006.01)*
**A61B 5/11** *(2006.01)*        **A61B 5/01** *(2006.01)*

(52) Cooperative Patent Classification (CPC):
**A61B 3/101; A61B 5/01; A61B 5/1103;
A61B 5/6821**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **10.09.2024  FI 20246104**

(71) Applicant: **IXI Eyewear Oy
02150 Espoo (FI)**

(72) Inventor: **Virtanen, Juha
00560 Helsinki (FI)**

(74) Representative: **Moosedog Oy
Kurjenmäenkatu 10 B 49
20700 Turku (FI)**

(54) **METHOD AND OPTICAL APPARATUS FOR CONTINUAL MONITORING TEAR FILM INTEGRITY OF EYE**

(57)    Disclosed is a method for continual monitoring of a tear film integrity of a user's eye (300). Method comprises determining a blink pattern of user's eye, using a means for detecting blinks of user's eye; measuring a temperature of an ocular surface of user's eye, at a set of at least two time instants within an inter-blink time interval of user's eye, using at least one temperature sensor (204A, 204B, 302); determining a rate of change in measured temperature for set of at least two time instants, based on a time interval between set of at least two time instants; comparing rate of change in measured temperature with a first threshold value; and when rate of change in measured temperature is greater than first threshold value: deeming tear film integrity of user's eye as broken.

FIG. 1

EP 4 706 501 A2

**Description**

TECHNICAL FIELD

The present disclosure relates to methods for continual monitoring of tear film integrity of user's eye. Moreover, the present disclosure relates to optical apparatuses.

BACKGROUND

Dry Eye Disease (DED) is a prevalent and often underdiagnosed eye condition affecting millions of people globally. The DED results from instability and hyperosmolarity of the tear film, leading to damage of ocular surface of the eye and associated symptoms like irritation, redness, and blurred vision. The said condition can significantly impair the quality of life, affecting daily activities such as reading, driving, and using digital devices. Accurate diagnosis and continuous monitoring of DED are crucial for effective management and treatment, but the heterogeneity of symptoms often means that many patients suffering from the DED remain unaware of their condition.

Existing solutions to address the problem of Dry Eye Disease are provided through various methods. In an existing solution, the DED is diagnosed by ophthalmologists using a combination of clinical assessments and specific tests. The two most prominent forms of DED are evaporative dry eye and aqueous-deficient dry eye (ADDE). The evaporative dry eye, caused by meibomian gland dysfunction, is often diagnosed using Tear Film Break-Up Time (TBUT) test, which assesses the integrity of the tear film. The Aqueous-deficient dry eye is diagnosed using the Schirmer's test, which measures tear production. Additionally, laboratory techniques such as laser interferometry estimate tear film thickness, while ocular surface thermography links tear film properties to surface temperature. However, these existing solutions are used intermittently and require clinical settings, limiting their practicality for continuous home-based monitoring. Despite the aforementioned existing solutions, significant challenges still persist. Tests like TBUT and Schirmer's are snapshot assessments that do not provide continuous monitoring, leading to potential delays in detecting tear film instability. Laser interferometry and thermography require specialized equipment and settings, making them impractical for everyday use. Moreover, commonly used indicators like blink rate are influenced by numerous factors and do not reliably correlate with tear film health.

Therefore, in light of the foregoing discussion, there exists a need to overcome the aforementioned drawbacks.

SUMMARY

The aim of the present disclosure is to provide a method and an optical apparatus to ensure a correct and regular assessment of a tear film's integrity to detect any changes that may indicate dry eye disease (DED) in a user's eye. The aim of the present disclosure is achieved by a method and an optical apparatus for continual monitoring of tear film integrity of user's eye as defined in the appended independent claims to which reference is made to. Advantageous features are set out in the appended dependent claims.

Throughout the description and claims of this specification, the words *"comprise", "include", "have",* and *"contain"* and variations of these words, for example *"comprising"* and *"comprises",* mean *"including but not limited to",* and do not exclude other components, items, integers or steps not explicitly disclosed also to be present. Moreover, the singular encompasses the plural unless the context otherwise requires. In particular, where the indefinite article is used, the specification is to be understood as contemplating plurality as well as singularity, unless the context requires otherwise.

BRIEF DESCRIPTION OF THE DRAWINGS

FIG. 1 is an illustration of a flowchart depicting steps of a method for continual monitoring of a tear film integrity of a user's eye, in accordance with an embodiment of the present disclosure;

FIG. 2 is a schematic illustration of an optical apparatus, in accordance with an embodiment of the present disclosure;

FIG. 3 is an illustration of a schematic illustration of a user's eye when an optical apparatus is in use, in accordance with an embodiment of the present disclosure; and

FIG. 4 is an illustration of a graphical representation of a first rate of change and a second rate of change in a measured temperature of an ocular surface of a user's eye, in accordance with an embodiment of the present disclosure.

DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

In a first aspect, the present disclosure provides a method for continual monitoring of a tear film integrity of a user's eye, the method comprising:

determining a blink pattern of the user's eye, using a means for detecting blinks of the user's eye;

measuring a temperature of an ocular surface of the user's eye, at a set of at least two time instants within an inter-blink time interval of the user's eye, using at least one temperature sensor;

determining a rate of change in the measured temperature for the set of at least two time instants, based on a time interval between the set of at least two time instants;

comparing the rate of change in the measured temperature with a first threshold value; and

when the rate of change in the measured temperature is greater than the first threshold value:

deeming the tear film integrity of the user's eye as broken.

The present disclosure provides an aforementioned method that is able monitor the tear film integrity of a user's eye accurately and continually across multiple time instants. Moreover, detection of the blink pattern helps to identify appropriate intervals for measuring the temperature of the ocular surface of the user's eye. Moreover, the method is able to detect even subtle changes in tear film stability that might indicate issues such as Dry Eye Disease (DED), before they become more severe, by comparing the rate of change in the measured temperature with a first threshold value. Furthermore, the method eliminates the need of self-reporting or manual monitoring by the user, providing an automated and objective way to monitor the blink pattern. The use of at least one temperature sensor allows for continuous and consistent data collection, reducing the possibility of human error. Furthermore, by focusing on the rate of temperature change rather than isolated temperature readings, the method is able to distinguish between normal variations and significant deviations indicative of a tear film issue. This can inform and alert the user about the immediate need to apply eye drops.

In a second aspect, the present disclosure provides a method for continuous monitoring of a tear film integrity of a user's eye, the method comprising:

determining a blink pattern of the user's eye, using a means for detecting blinks of the user's eye;

measuring a temperature of an ocular surface of the user's eye, at a plurality of time instants during a time duration, wherein said time instant lies within an inter-blink time interval of the user's eye, using at least one temperature sensor;

determining an average temperature of the ocular surface of the user's eye for the time duration, based on the measured temperature of the ocular surface at the plurality of time instants;

comparing the average temperature of the ocular surface of the user's eye for the time duration with a second threshold value; and

when the average temperature of the ocular surface of the user's eye for the time duration is greater than the second threshold value:

deeming the tear film integrity of the user's eye as broken.

Herein, the method is able to detect even minor deviations in the ocular surface temperature that are indicative of the issues such as Dry Eye Disease, before they become more severe, by comparing the average temperature with a second threshold value. Furthermore, the method eliminates the need of self-reporting or manual monitoring by the user, providing an automated and objective way to provide indication of tear film breakdown before appearance of symptoms. The use of at least one temperature sensor allows for continuous and consistent data collection, reducing the possibility of human error. Furthermore, by focusing on the average temperature of the ocular surface of the user's eye rather than isolated temperature readings, the method is able to distinguish between normal variations and significant deviations indicative of the tear film issue. This can reduce false positives and improve the reliability of the assessment.

In yet an one aspect, the present disclosure provides an optical apparatus comprising:

a frame;

at least one temperature sensor arranged on the frame, wherein when the optical apparatus is in use, the at least one temperature sensor faces a user's eye; and

at least one processor coupled to the at least one temperature sensor, wherein the at least one processor is configured to:

determine a blink pattern of the user's eye, using a means for detecting blinks of the user's eye;
control the at least one temperature sensor to measure a temperature of an ocular surface of the user's eye, at a set of at least two time instants within an inter-blink time interval of the user's eye,;
determine a rate of change in the measured temperature for the set of at least two time instants, based on a time interval between the set of at least two time instants;
compare the rate of change in the measured temperature with a first threshold value,; and
when the rate of change in the measured temperature is greater than the first threshold value,:
deem the tear film integrity of the user's eye as broken.

In a third aspect, the present disclosure provides an optical apparatus comprising:

a frame;
at least one temperature sensor arranged on the frame, wherein when the optical apparatus is in use, the at least one temperature sensor faces a user's eye; and
at least one processor coupled to the at least one temperature sensor, wherein the at least one processor is configured to:

determine a blink pattern of the user's eye, using a means for detecting blinks of the user's eye;
control the at least one temperature sensor to measure a temperature of an ocular surface of the user's eye, at a set of at least two time instants within an inter-blink time interval of the user's eye, wherein the set of at least two time instants is sets of at least two time instants and each set of at least two time instants amongst the sets of at least two time instants lie within a corresponding inter-blink time interval of the user's eyes for a corresponding blink of the user's eye;
determine a rate of change in the measured temperature for the set of at least two time instants, based on a time interval between the set of at least two time instants, wherein the set of at least two time instants is each set of at least two time instants amongst the sets of at least two time instants;
determine an average rate of change in the measured temperature, based on the rate of change in the measured temperature for each set of at least two time instants amongst the sets of at least time instants;
compare the rate of change in the measured temperature with a first threshold value, wherein the rate of change is the average rate of change; and
when the rate of change in the measured temperature is greater than the first threshold value, wherein the rate of change is the average rate of change:
deem the tear film integrity of the user's eye as broken.

The present disclosure provides an aforementioned optical apparatus that is able to monitor the temperature of the ocular surface of the user's eye effectively and continually for the set of at least two time instants. Moreover, the at least one temperature sensor in the optical apparatus provides a simple and convenient way for estimating the temperature of the ocular surface of the user's eye in a non-contact and easy to implement manner. Advantageously, the measurement of ocular surface temperature when used to assess the user's eye condition, provides details on the tear film integrity with precision, accuracy, and allows measurement of individualized data. Furthermore, the optical apparatus eliminates the need for user self-reporting or manual monitoring, providing an automated and objective way to provide indication of tear film breakdown before appearance of the symptoms. Furthermore, use of the at least one processor allows for continuous and consistent data collection, reducing the possibility of human error.

In an one other aspect, the present disclosure provides an optical apparatus comprising:

a frame;
at least one temperature sensor arranged on the frame, wherein when the optical apparatus is in use, the at least one temperature sensor faces a user's eye; and
at least one processor coupled to the at least one temperature sensor, wherein the at least one processor is configured to:

determine a blink pattern of the user's eye, using a means for detecting blinks of the user's eye;
control the at least one temperature sensor to measure a temperature of an ocular surface of the user's eye, at a plurality of time instants during a time duration, wherein said time instant lies within an inter-blink time interval of the user's eye,;
determine an average temperature of the ocular surface of the user's eye for the time duration, based on the measured temperature of the ocular surface at the plurality of time instants;
compare the average temperature of the ocular surface of the user's eye for the time duration with a second threshold value; and
when the average temperature of the ocular surface of the user's eye for the time duration is greater than the second threshold value:
deem the tear film integrity of the user's eye as broken.

In a fourth aspect, the present disclosure provides an optical apparatus comprising:

a frame;
at least one temperature sensor arranged on the frame, wherein when the optical apparatus is in use, the at least one temperature sensor faces a user's eye; and
at least one processor coupled to the at least one temperature sensor, wherein the at least one processor is configured to:

determine a blink pattern of the user's eye, using a means for detecting blinks of the user's eye;
control the at least one temperature sensor to measure a temperature of an ocular surface of the user's eye, at a plurality of time instants during a time duration, wherein said time instant lies within an inter-blink time interval of the user's eye, and wherein the measured temperature of the ocular surface of user's eye is adjusted based on at least one of: a blink rate, a blink duration, an ambient temperature, an ambient lighting, a type of environment, a type of activity associated with the user's eye;
determine an average temperature of the ocular surface of the user's eye for the time duration, based on the measured temperature of the ocular surface at the plurality of time instants;
compare the average temperature of the ocular surface of the user's eye for the time duration with a second threshold value; and
when the average temperature of the ocular surface of the user's eye for the time duration is greater than the second threshold value:
deem the tear film integrity of the user's eye as broken.

The present disclosure provides an aforementioned optical apparatus that is able to monitor the temperature of the ocular surface of the user's eye effectively and continuously across plurality of time instants. Moreover, the at least one temperature sensor in the optical apparatus provides a simple and convenient way for estimating the temperature of the ocular surface of the user's eye in a non-contact and easy to implement manner. Furthermore, the optical apparatus eliminates the need for user self-reporting or manual monitoring, providing an automated and objective way to provide indication of tear film breakdown before appearance of the symptoms. Furthermore, change in the ocular surface temperature of the user's eye reflects the rate of evaporation and subsequently provide insights about the thickness of the tear film. Furthermore, use of the at least one processor allows for continuous and consistent data collection, reducing the possibility of human error. Furthermore, beneficially, the aforementioned optical apparatus adjusts the measured temperature while compensating for individual factors (such as blink rate and duration) and environmental influences (such as ambient temperature and lighting). Such adjustment provides a more accurate assessment of the true ocular surface condition and reduces diagnostic errors caused by external or transient influences, leading to more reliable and clinically relevant evaluations of tear film integrity.

Throughout the present disclosure, the term *"continual monitoring"* refers to process of continuously detecting and tracking a condition of the tear film integrity over an extended period. Notably, the continual monitoring of the tear film integrity enables to detect any significant changes in the tear film integrity of the user's eye which indicates towards the tear film integrity to be broken. Throughout the present disclosure, the term *"tear film integrity"* refers to stability, consistency and overall health of a thin layer of fluid that covers the surface of the user's eye. Typically, the tear film is composed of three main components, lipid layer, aqueous layer and the mucin layer. Moreover, the tear film integrity depends on maintaining an appropriate thickness, a uniform distribution, and a protective function of the tear film to prevent evaporation and ensure clear vision in the user's eye. Notably, continual monitoring of the tear film integrity in the user's eye ensures detection of any changes or abnormalities in the tear film integrity as they occur, which is crucial for treating eye conditions and ocular diseases such as glaucoma, dry eye disease and the like. Moreover, a significant number of users fail to know that they are suffering from the said ocular diseases. Thus, the continual monitoring of the tear film integrity facilitates in promoting early detection and compliance with required treatment. Advantageously, by monitoring the tear film integrity continually, the method can detect early signs of deterioration of the tear film, allowing for timely interventions to prevent discomfort and potential long-term complications associated with the ocular diseases.

Throughout the present disclosure, the term *"blink pattern"* refers to a specific pattern or behavior associated with the user's blinking activity. Typically, the blink pattern may include blink rate, blink duration, blink intensity, blink sequence and the like. The term *"blinks"* refers to rapid closing and opening of eyelids of the user's eye. Typically, the blinks provide several essentials functions such as lubrication, protection and oxygenation to the user's eye. Moreover, the blink pattern can vary based on individual differences of the user, environmental conditions and the presence of eye conditions such as DED. It will be appreciated that detecting the blink pattern of the user's eye enables to accurately determine when the user's eye blinks, and subsequently, the inter-blink time interval is accurately determined. In this context, the term *"blink rate"* refers to how frequently the eyes blink, and blink rate is measured corresponding to a unit time. Similarly, the term *"blink duration"* refers to a time interval for which the eyelids remain closed during a single blink. Typically, the blink duration may be short, medium, or long blinks depending on the said time interval. Similarly, the term *"blink intensity"* refers to a degree of completeness of a blink such as how fully and firmly the eyelids close during a blink and the term *"blink sequence"* refers to the order and timing of blinks over a monitoring time interval. The blink sequence may be analyzed to identify irregularities such as clusters of rapid blinks or extended pauses that could influence ocular surface temperature and tear film integrity assessment.

It may be appreciated that detection of blink pattern may be achieved by a suitable means which may be, but is not limited to, at least one of: an Infrared (IR) camera, an IR sensor, a photodiode, a light sensor, an electromyography (EMG) sensor and the likes.

Throughout the present disclosure, the term *"ocular surface of the user's* eye" refers to a surface around the user's eye that comprises at least one of: a corneal surface of the user's eye, a surrounding skin surface of the corneal surface, an eyelid surface. Herein, the term *"temperature"* refers to an internal temperature of the ocular surface of the user's eye. The temperature of the ocular surface of the user's eye being in a range of 32°C and 36°C is considered to be of a normal level. Notably, measuring the temperature of the ocular surface of the user's eye is crucial for monitoring the tear film integrity of the user's eye, as significant changes in the temperature of the ocular surface are indicative of the rate of tear evaporation and health of the tear film of the user's eye. It will be appreciated that the temperature of the ocular surface of the user's eye is measured by determining an average of measured temperature of at least one point on the ocular surface of the user's eye. Notably, the temperature of the ocular surface of the user's eye is measured using the at least one temperature sensor. Throughout the present disclosure, the term *"temperature sensor"* refers to a sensing device that is able to sense a temperature of the ocular surface of the user's eye.

Throughout the present disclosure, the term *"time instant"* refers to a particular moment of time at which the temperature of the ocular surface of the user's eye is measured by the at least one temperature sensor. Notably, the temperature of the ocular surface of the user's eye being measured at the set of at least two time instants implies that the temperature of the ocular surface is measured at two or more time instants within the inter-blink time interval for a given blink of the user's eye. Throughout the present disclosure, the term *"inter-blink time interval"* refers to a time-interval between two successive blinks of the user's eye. Notably, the inter-blink time interval starts when the eyelid opens after a blink and ends when the eyelid closes for the next blink. It will be appreciated that the set of at least two time instants being within the inter-blink time interval of the user's eye ensures that the temperature of the ocular surface of the user's eye is measured at those moments when the use's eye is open, and thus, the temperature of the ocular surface is measured accurately.

Optionally, the time interval between the set of at least two time instants is of at least 10 seconds. In this regard, the time interval between the set of at least two time instants is in the range of 10 seconds to 20 seconds. Optionally, the time interval between the set of at least two time instants may be in the range of 10, 12, 14, 16 or 18 seconds up to 12, 14, 16, 18 or 20 seconds. Notably, by ensuring the time interval of at least 10 seconds between the set of at least two instants, the rate of change in the measured temperature, that occurs over a significant period of time, is determined, which is likely to be more reliable to monitor the tear film integrity. A technical effect is that the measured temperature data is less susceptible to noise and short-term variations, resulting in more reliable and accurate values of the temperature of the ocular surface of the user's eye.

It will be appreciated that by measuring the ocular surface temperature at the set of at least two time instants within the inter-blink time interval, the method can analyze changes in the temperature that occur between the blinks of the user's eye, providing valuable information about the tear film's stability. Moreover, the at least one temperature sensor is used for measuring the temperature of the ocular surface of the user's eye at the set of at least two time instants, which implies that the temperature of the ocular surface of the user's eye is measured at each time instant amongst the at least two time instants within the inter-blink time interval of the user's eye. For example, the at least one temperature sensor is at least one of: thermocouples, resistance temperature detectors, thermistors, or infrared sensors. In an implementation, the at least one temperature sensor comprises a plurality of temperature sensors which enables coverage of a broader area of the ocular surface of the user's eye.

Throughout the present disclosure, the term *"time interval"* refers to a period between two specific moments of time at which the temperature measurements are taken within the inter-blink period. Notably, the time interval is an elapsed time between the set of at least two time instants at which temperature readings of the ocular surface of the user's eye are recorded and the temperature readings are used to calculate the rate of change in the measured temperature for the pair of at least two time instants. Moreover, the rate of change in the measured temperature is able to provide insights about the tear evaporation and the tear film dynamics. A higher rate of change in the measured temperature may indicate a thinner tear film and higher evaporation rates, which are characteristics of the DED. Furthermore, the temperature measurements for the set of at least two time instants are taken within the inter-blink interval using the temperature sensor. The duration between the temperature measurements within the time interval is recorded. The rate of change of the measured temperature is calculated by dividing the difference in temperature readings at the at least two time instants by the time interval. Mathematically, the rate of change of the measured temperature is expressed as:

$$\text{Rate of Change} = \Delta T / \Delta t,$$

where $\Delta T$ is the change in the measured temperature and $\Delta t$ is the time interval between the measurements at the at least two time instants.

Advantageously, the ability to monitor and analyze the rate of change of the measured temperature for the pair of set of at least two intervals allows for early detection of abnormalities in tear film dynamics.

Throughout the present disclosure, the term *"first threshold value"* refers to a threshold change in the temperature value of the ocular surface of the user's eye measured at the at least two time instants. Notably, the first threshold value acts as a predetermined reference point to evaluate and compare the rate of change in the measured temperature. Moreover, the first threshold value is set based on empirical data or clinical studies and serves as a critical parameter for diagnosing the integrity of the tear film. Moreover, the calculated rate of change in the measured temperature is then compared to the first threshold value to check whether the rate of change in the measured temperature is above, below, or within the threshold range. Advantageously, using the first threshold value ensures an objective and standardized method for evaluating the rate of change in ocular surface temperature (OST), reducing variability and subjectivity in assessments. Furthermore, the comparison can be tailored to individual users by adjusting the threshold value based on personal ocular characteristics and clinical history of the user.

Optionally, the first threshold value is of 0.04° Celsius per second (°C/s). In this regard, the first threshold value is in range of 0.00 to 0.05°C/s. optionally, the first threshold value may be in the range of 0.00, 0.01, 0.02, 0.03 or 0.04°C/s up to 0.01, 0.02, 0.03, 0.04 or 0.05°C/s. Notably, setting the first threshold value at 0.04°C/s provides a clear, standardized benchmark for assessing the tear film integrity. Moreover, the specific threshold value is chosen based on clinical research and empirical data to effectively distinguish between normal and abnormal rates of OST change, which are indicative of healthy or compromised tear film. A technical effect of choosing the aforementioned value as the first threshold value provides an objective criterion for evaluation, ensuring consistency in the accurate detection of the tear film integrity across different users and situations, thereby improving eye health outcomes.

Throughout the present disclosure, it will be appreciated that if the rate of change in the measured temperature exceeds the first threshold value, the tear film integrity of the user's eye is considered to be compromised or broken. Notably, deeming the tear film integrity as broken when the rate of change exceeds the first threshold value is crucial for diagnosing the dry eye disease (DED) and other related conditions. Moreover, a high rate of change in the measured temperature suggests rapid tear film evaporation or inadequate tear film production, which are key indicators of compromised tear film integrity. Furthermore, the ability to deem the tear film integrity as broken when the rate of change is too high allows for immediate action, such as alerting the user to apply eye drops or seek medical advice even when the symptoms of DED are not clearly visible to the user.

Furthermore, by compensating for individual factors (such as blink rate, blink duration, type of activity), the adjusted temperature provides a more accurate representation of the true ocular surface condition, independent of transient environmental fluctuations or user behavior. Adjusting the measurement based on personal parameters such as blink rate and blink duration tailors the temperature assessment uniquely to the individual user, allowing more precise identification of ocular conditions like tear film instability. Accounting for the aforementioned individual factors significantly reduces diagnostic errors caused by external or transient influences, leading to more reliable and clinically relevant evaluations of tear film integrity.

Optionally, the method further comprises:

measuring the temperature of the ocular surface of the user's eye at sets of at least two time instants, wherein each set of at least two time instants amongst the sets of at least two time instants lie within a corresponding inter-blink time interval of the user's eyes for a corresponding blink of the user's eye;

determining the rate of change in the measured temperature for each set of at least two time instants amongst the sets of at least two time instants;

determining an average rate of change in the measured temperature, based on the rate of change in the measured temperature for each set of at least two time instants amongst the sets of at least time instants;

comparing the average rate of change in the measured temperature with the first threshold value; and

when the average rate of change in the measured temperature is greater than the first threshold value:
deeming the tear film integrity of the user's eye as broken.

In this regard, the term *"corresponding inter-blink time interval"* refers to the inter-blink time interval between two successive corresponding blinks of the set of at least two time instants amongst the set of at least two time instants for which the temperature of the ocular surface of the user's eye is measured. Notably, each inter-blink time interval is unique and directly related to the blink pattern of the user. Throughout the present disclosure, the term *"corresponding blink"* refers to a particular blink of the user's eye associated with the set of at least two time instants amongst the set of at least two time instants for which the ocular surface temperature of the user's eye is measured. Notably, the temperature of the ocular surface of the user's eye at sets of at least two time instants is measured to accurately assess changes in the measured temperature that are influenced by the blink cycle. Moreover, measuring the OST within the corresponding inter-blink time intervals ensures that the temperature data reflects changes that occur due to the blinking and provides a reliable basis for evaluating the tear film integrity.

The term *"average rate of change"* refers to a mean value of the rates at which the ocular surface temperature of the user's eye changes over the corresponding inter-blink time intervals. Notably, the average rate of change is determined based on the temperature measurements taken at each set of at least two time instants amongst the sets of at least time instants within the corresponding inter-blink time intervals. Furthermore, the average rate of change is computed by averaging the individual rate of change in the measured temperature, providing a single representative value that reflects the overall trend in the temperature change over the observation period. A technical effect is that averaging the rate of change of the measured temperature mitigates the effect of short-term fluctuations and environmental factors, providing a more accurate assessment of the user's eye conditions. Furthermore, the method offers a more precise indication of the tear film integrity compared to singlepoint measurements and accounts for individual blink patterns, ensuring that the monitoring is personalized and accurate.

Moreover, by averaging the rate of change of the measured temperature the effect of short-term fluctuations and environmental factors can be effectively mitigated, providing a more accurate assessment of the user's eye conditions. Furthermore, a more precise indication of the tear film integrity compared to single point measurements is obtained, and such measurement accounts for individual blink patterns, ensuring that the monitoring is personalized and accurate. Separate blink detection makes it possible to average out ocular surface temperature changes not visible in a raw IR thermographic waveform. This, in turn, enables the integration of accurate monitoring capabilities into a smart eyewear (such as a smart eyeglass).

Optionally, the method further comprises:

measuring the temperature of the ocular surface of the user's eye at sets of at least two time instants, wherein each set of at least two time instants amongst the sets of at least two time instants lie within a corresponding inter-blink time interval of the user's eyes for a corresponding blink of the user's eye whose blink duration lies within a predefined range;

determining the rate of change in the measured temperature for each set of at least two time instants amongst the sets of at least two time instants;

determining an average rate of change in the measured temperature, based on the rate of change in the measured temperature for each set of at least two time instants amongst the sets of at least time instants;

comparing the average rate of change in the measured temperature with the first threshold value; and

when the average rate of change in the measured temperature is greater than the first threshold value:
deeming the tear film integrity of the user's eye as broken.

In this regard, the term *"predefined range"* refers to a specific interval of time that is set in advance and within which the blink duration of a corresponding blink must fall for the temperature measurements to be considered valid. Typically, the predefined range is determined based on empirical data or clinical standards. The term *"blink duration"* refers to the total time taken for a single blink of the user's eye, measured from the moment the eyelid begins to close until the eyelid returns to its fully open position. Notably, the blink duration is used to filter out blinks that are too short or too long and select specific blinks for which the ocular surface temperature measurements are taken, ensuring that the blinks within a consistent and the predefined range are analyzed. Moreover, the ocular surface temperature is measured at least two times within each inter-blink interval for the blinks that duration falls within the predefined range. Subsequently, for each set of temperature measurements, the rate of change in the measured temperature is determined. The rates of change for multiple sets are averaged to obtain a more stable and reliable indicator. The average rate of change is compared against the first threshold value. Consequently, if the average rate of change exceeds the first threshold, the tear film integrity is deemed to be broken, indicating potential the dry eye disease. A technical effect is that using a predefined range for blink durations ensures that the temperature measurements are consistent and comparable, reducing variability and improving the reliability of the diagnosis. Additionally, the ability to detect tear film breakdown before symptoms appear allows for early intervention, potentially preventing more severe dry eye complications.

Optionally, the method further comprises:

measuring the temperature of the ocular surface of the user's eye at sets of at least two time instants, wherein each set of at least two time instants amongst the sets of at least two time instants lie within a corresponding inter-blink time interval of the user's eyes for a corresponding blink of the user's eye;

associating each set of at least two time instants amongst the sets of at least two time instants with a corresponding category, based on a blink duration of the corresponding blink of the user's eye;

determining the rate of change in the measured temperature for each set of at least two time instants amongst the sets of at least two time instants;

normalizing the determined rate of change of the measured temperature for each set of least two time instants, based on the determined corresponding category associated with said set of at least two time instants;

determining an average rate of change in the measured temperature, based on the normalized rate of change in the measured temperature for each set of at least two time instants amongst the sets of at least time instants;

comparing the average rate of change in the measured temperature with the first threshold value; and

when the average rate of change in the measured temperature is greater than the first threshold value:
deeming the tear film integrity of the user's eye as broken.

In this regard, the term *"corresponding category"* refers to a classification or group assigned to each set of at least two time instants. Notably, the corresponding category is determined based on the blink duration of the corresponding blink within the corresponding inter-blink time interval of the user's eye. Each blink duration falls within a predefined range, that represents a category, allowing for more precise analysis and comparison of the measured temperatures. Moreover, each set of at least two time instants of temperature measurement is associated with a category based on the recorded blink duration i.e., the blink duration of the corresponding blink (which may include, for example, short blinks lasting less than 100 milliseconds, medium blinks lasting between 100 and 300 milliseconds, or long blinks lasting more than 300 milliseconds). For example, the corresponding blinks may be categorized into short, medium, or long durations. Subsequently, the temperature data from each set of at least two time instants is then associated with the corresponding category based on the blink duration. Advantageously, the categorization allows for consistent comparison and analysis across different blinks, reducing the impact of irregularities and anomalies in the blink patterns. The term *"normalizing"* refers to a process of adjusting the rate of change of the measured temperature based on the characteristics of the corresponding category. For example, if a certain category typically has a higher baseline rate of change, the measured rate of change for that category might be adjusted downward to reflect the baseline. Notably, the normalization is done based on the corresponding category associated with each set of at least two time instants. Furthermore, the normalization is necessary to account for variations in blink patterns that can affect the rate of temperature change. The corresponding category of blinks can have different baseline rates of temperature change. By normalizing the data, the method ensures that the rate of change is adjusted to a common scale, making it possible to compare and analyze the temperature changes accurately across different blink categories. A technical effect is that the normalization of the rate of change of the measured temperature ensures that the method can more precisely identify deviations from normal behavior, improving the accuracy of diagnosing tear film integrity issues. Beneficially, normalization allows for consistent and reliable comparison across different blink patterns, further improving the accuracy of the assessment. Additionally, the normalization process adapts the analysis to the individual user's blink patterns, making the monitoring more personalized and effective.

The present disclosure also relates to the method of the second aspect as described above. Various embodiments and variants disclosed above, with respect to the aforementioned method of continuous monitoring of a tear film integrity of the first aspect apply mutatis mutandis to the method of the second aspect.

In the second aspect, the term *"time duration"* refers to a predefined duration of time for which the temperature of the ocular surface of the user's eye is to be tracked. Typically, the time duration can vary, depending on the user habits or specific monitoring protocols. Notably, the time duration encompasses the inter-blink time interval between consecutive blinks of the user's eye. In the second aspect, optionally, the time duration is one of: 30 minutes, 1 hour, 5 hours. In this regard, the time duration is predetermined and can be set to specific lengths, such as 30 minutes, 1 hour, or 5 hours, to facilitate systematic and consistent monitoring of the tear film integrity over a specified period. It will be appreciated that the time duration may be in the range of 1, 5, 10, 15, 20 or 25 minutes up to 5, 10, 15, 20, 25 or 30 min. Optionally, the time duration may be in the range of 1, 10, 20, 30, 40 or 50 min up to 10, 20, 30, 40, 50 or 1 hour. Optionally, the time duration may be in the range of 1, 2, 3 or 4 hours up to 2, 3, 4 or 5 hours. Notably, the time duration is set to one of: 30 minutes, 1 hour, 5 hours to provide flexibility in monitoring the tear film integrity based on different user needs or clinical requirements. A technical effect is that different durations allow for short-term, mid-term, and long-term assessments, which can be useful for various diagnostic and therapeutic purposes such as longer durations (such as 5 hours) can provide a more detailed and comprehensive picture of the tear film dynamics, which is useful for chronic monitoring and indepth analysis and shorter durations (like 30 minutes) can be used for quick assessments, making the process more efficient and less resourceintensive.

In the second aspect, the term *"plurality of time instants"* refers to multiple different instances of time spread across the entire period of the time duration. Notably, the plurality of time instants lies within the inter-blink time interval of the user's eye. Subsequently, measuring the temperature of the ocular surface of the user's eye at the plurality of time instants enables accurate and reliable tracking of the temperature of the ocular surface of the user's eye over the entire period of the time duration. Moreover, measuring the temperature of the ocular surface of the user's eye at the plurality of time instants within the time duration, enables collection of the data points by the at least one temperature sensor required for generating a temperature trend.

The term *"average temperature"* refers to a calculated mean temperature of the ocular surface of the user's eye based on the measured temperature of the ocular surface at the plurality of time instants over the time duration. Typically, the average temperature of the ocular surface of the user's eye is determined by summing the individual temperature readings taken at the plurality of time instants and then dividing this sum by the number of readings. Advantageously, the average temperature provides a stable and representative value of the ocular surface temperature over the time duration, reducing the impact of short-term fluctuations and outliers. Moreover, the average temperature facilitates a baseline or reference value that reflects the general condition of the ocular surface over the time duration.

In the second aspect, the term *"second threshold value"* refers to a threshold change in the temperature value of the ocular surface of the user's eye measured at the plurality of time instants. Notably, the second threshold value acts as a predetermined reference point to evaluate and compare the average temperature of the ocular surface of the user's eye. Moreover, the first threshold value is set based on empirical data or clinical studies and serves as a critical parameter for diagnosing the integrity of the tear film. Moreover, the average temperature of the ocular surface of user's eye based on the measured temperature is then compared to the second threshold value to check whether the rate of change in the measured temperature is above, below, or within the threshold range. Advantageously, using the second threshold value ensures an objective and standardized method for evaluating the rate of change in OST, reducing variability and subjectivity in assessments.

In the second aspect, optionally, the second threshold value is determined from a predetermined average temperature of the ocular surface of the user's eye for a duration of at least 2 hours. In this regard, the term *"predetermined average temperature"* refers to a previously established mean temperature value of the ocular surface of the user's eye, which is calculated based on the temperature measurements taken over a specified period of at least 2 hours. Notably, the predetermined average temperature value serves as a baseline or reference point to establish the second threshold value for assessing tear film integrity.

Moreover, predetermined average temperature represents the expected temperature condition of the ocular surface under normal circumstances. The predetermined average temperature is obtained by taking multiple temperature readings over a continuous period of at least 2 hours, calculating the average of these readings, and then using this average as a reference. For example, the ocular surface temperature is measured every 10 minutes over a 2-hour period, resulting in the following readings: 31.8°C, 31.9°C, 32.0°C, 31.7°C, 31.9°C, 32.1°C, 31.8°C, 31.9°C, 32.0°C, 31.8°C, 31.9°C, and 32.0°C. The predetermined average temperature would be calculated as follows:

$$\text{Predetermined Average Temperature} =$$
$$(31.8+31.9+32.0+31.7+31.9+32.1+31.8+31.9+32.0+31.8+31.9+32.0 12)/12 = 31.89°C.$$

A technical effect of using the predetermined average temperature helps in setting precise and reliable threshold values for comparison during real-time monitoring. This enables the detection of deviations from normal temperature patterns, which can indicate that the tear film integrity is broken.

In the second aspect, optionally, the method further comprises: adjusting the measured temperature of the ocular surface of user's eye based on at least one of: a blink rate, a blink duration, an ambient temperature, an ambient lighting, a type of environment, a type of activity associated with the user's eye.

In this regard, the term *"blink rate"* refers to the frequency at which the user's eye blinks over a specified period. Typically, the blink rate is measured in blinks per minute (BPM). Notably, the blink rate is a significant factor in assessing ocular surface health and tear film stability. Whilst measuring the temperature of the ocular surface, the blink rate can influence the accuracy and interpretation of the temperature readings. For instance, a higher blink rate might result in more frequent rewetting of the eye surface, potentially affecting temperature stability. Therefore, adjusting the temperature measurements based on the blink rate can help in obtaining more precise and meaningful data regarding the ocular surface condition. The term *"blink duration"* refers to a length of time during which the eyelids of the user's eye remain closed during a single blink cycle. Typically, the blink duration is measured in milliseconds (ms). Notably, the blink duration is measured using high-speed video recording or specialized sensors that detect the opening and closing of the eyelids. The adjustment in the blink duration is important in assessing ocular surface health and tear film dynamics. A longer blink duration can lead to more effective spreading of the tear film over the ocular surface, while shorter blink durations may result in incomplete or insufficient tear film distribution. The term *"ambient temperature"* refers to the temperature of a surrounding environment in which the user's eye is situated. Typically, the ambient temperature can significantly influence the temperature of the ocular surface. For example, in colder environments, the ocular surface temperature may drop, while in warmer environments, it may rise. Adjusting the measured ocular surface temperature based on the ambient temperature helps in isolating the actual ocular surface conditions from environmental influences. The term *"type of environment"* refers to specific characteristics and conditions of the surroundings in which the user is located. Typically, the type of environment may include factors such as humidity levels, air quality, presence of wind or drafts, and overall climatic conditions. Different environments can affect the ocular surface temperature and overall eye health. For instance, dry or windy environments can lead to faster evaporation of the tear film, influencing the temperature and integrity of the ocular surface. By adjusting the measured temperature of the ocular surface based on the type of environment, the method can provide a more accurate assessment of the eye's condition, considering the external factors that might influence the readings. The term *"type of activity"* refers to that specific task or action the user is engaged in that can influence their blink rate, blink duration, and overall eye condition. Typically, the type of activity may include activities such as reading, using digital devices, driving, outdoor sports, or any other activity that affects eye usage and strain. Notably, different activities can have varying impacts on the blink patterns and the ocular surface temperature. For example, prolonged screen time typically reduces the blink rate and increases the blink duration, which can lead to dry eye symptoms. A technical effect is that the temperature of the ocular surface can be influenced by several external and internal factors. Adjusting the measured temperature based on these variables ensures that the data reflects the true state of the user's eye. Additionally, by considering the said factors, the method provides a personalized assessment that is more accurate for each individual. In other words, adjusting the measured temperature in the aforementioned manner (i.e., based on various factors as mentioned above) provides a more accurate and personalized assessment of tear film integrity, compensating for individual and environmental factors that may affect ocular surface temperature.

Moreover, the average temperature of the ocular surface of the user's eye is compared with the second threshold value. If the average temperature of the ocular surface of the user's eye exceeds the second threshold value, the tear film integrity is deemed to be compromised or broken.

The present disclosure also relates to the optical apparatus as described above. Various embodiments and variants disclosed above, with respect to the aforementioned method of continuous monitoring of a tear film integrity apply mutatis mutandis to the optical apparatus.

Throughout the present disclosure, the term *"optical apparatus"* refers to an arrangement of electrical and/or mechanical components that is worn around eyes of the user. Optionally, the optical apparatus (practically, a smart eyewear) is employed, for example, to correct a vision disorder, including but not limited to, myopia, hypermetropia, astigmatism, presbyopia and the like, such as, by enabling appropriate refraction, reflection, diffraction or transmission of light towards the eyes of the user wearing the optical apparatus. Beneficially, the optical apparatus is used to provide protection to the eyes of the user from ambient conditions such as sunlight, ultraviolet radiation, blue light associated with computing devices, dust, chemical fumes, and such like. Moreover, the optical apparatus enables display of information to the eyes of the user. Throughout the present disclosure, the term "frame" refers to a mechanical component of the eyewear apparatus that acts as a stable base to enable the user to wear the optical apparatus around the eyes. It will be appreciated that the frame is fabricated, for example, with cellulose acetate, propionate plastic, nylon-based plastic, polyamide, epoxy resin, carbon fiber, metal, metal alloy and the like.

Throughout the present disclosure, the term *"processor"* refers to a computational element that is operable to execute instructions of the optical apparatus. Examples of the at least one processor include, but are not limited to, a microprocessor, a microcontroller, a complex instruction set computing (CISC) microprocessor, a reduced instruction set (RISC) microprocessor, a very long instruction word (VLIW) microprocessor, or any other type of processing circuit. Furthermore, the at least one processor may refer to one or more individual servers, processing devices and various elements associated with a processing device that may be shared by other processing devices. Additionally, one or more individual processors, processing devices and elements are arranged in various architectures for responding to and processing the instructions that execute the instructions of the optical apparatus. Optionally, the at least one processor is coupled to the at least one temperature sensor in a wired or wireless manner. Notably, prior to estimating the temperature of the ocular surface of the user's eye, the at least one processor is configured to receive sensor data from the at least one temperature sensor, the sensor data being indicative of the measured temperature of the plurality of time instants. Moreover, the at least one processor analyzes the sensor data to determine the rate of change in the measured temperature, representing how the rate of change in the measured temperature of the ocular surface of user's eye is greater than the first threshold value and determines that the tear film integrity of the user's eye as broken.

Optionally, the at least one processor is further configured to:

measure the temperature of the ocular surface of the user's eye at sets of at least two time instants, wherein each set of at least two time instants amongst the sets of at least two time instants lie within a corresponding inter-blink time interval of the user's eyes for a corresponding blink of the user's eye;

determine the rate of change in the measured temperature for each set of at least two time instants amongst the sets of at least two time instants;

determine an average rate of change in the measured temperature, based on the rate of change in the measured temperature for each set of at least two time instants amongst the sets of at least time instants;

compare the average rate of change in the measured temperature with the first threshold value; and

when the average rate of change in the measured temperature is greater than the first threshold value:
deem the tear film integrity of the user's eye as broken.

Optionally, the at least one processor is further configured to:

measuring the temperature of the ocular surface of the user's eye at sets of at least two time instants, wherein each set of at least two time instants amongst the sets of at least two time instants lie within a corresponding inter-blink time interval of the user's eyes for a corresponding blink of the user's eye , wherein the user's eye blink duration lies within a predefined range;
determining the rate of change in the measured temperature for each set of at least two time instants amongst the sets of at least two time instants;
determining an average rate of change in the measured temperature, based on the rate of change in the measured temperature for each set of at least two time instants amongst the sets of at least time instants;
comparing the average rate of change in the measured temperature with the first threshold value; and
when the average rate of change in the measured temperature is greater than the first threshold value:
deeming the tear film integrity of the user's eye as broken.

Optionally, the at least one processor is further configured to:

measure the temperature of the ocular surface of the user's eye at sets of at least two time instants, wherein each set of at least two time instants amongst the sets of at least two time instants lie within a corresponding inter-blink time interval of the user's eyes for a corresponding blink of the user's eye;
associate each set of at least two time instants amongst the sets of at least two time instants with a corresponding category, based on a blink duration of the corresponding blink of the user's eye;
determine the rate of change in the measured temperature for each set of at least two time instants amongst the sets of at least two time instants;
determine an average rate of change in the measured temperature, based on the rate of change in the measured temperature for each set of at least two time instants amongst the sets of pairs of time instants, wherein the rate of change is the normalized rate of change;
compare the average rate of change in the measured temperature with the first threshold value; and
when the average rate of change in the measured temperature is greater than the first threshold value:
deem the tear film integrity of the user's eye as broken.

It may be appreciated that the at least one processor is configured to adjust the measured temperature based on various individual factors (such as blink rate and duration) and environmental influences (such as the ambient temperature, the ambient lighting, the type of environment). This adjustment enables the optical apparatus to provide a more accurate and personalized assessment of tear film integrity, compensating for such individual and environmental factors that may affect ocular surface temperature. Moreover, the at least one processor associates each set of time instants with a category based on blink duration, and normalize the rate of change accordingly to obtain consistent and reliable comparison across different blink patterns, further improving the accuracy of the assessment.

The present disclosure also relates to the optical apparatus as described above. Various embodiments and variants disclosed above, with respect to the aforementioned method for continuous monitoring of a tear film integrity and the aforementioned optical apparatus, apply mutatis mutandis to the optical apparatus.

Optionally, the at least one processor is further configured to:

measure the temperature of the ocular surface of the user's eye at sets of at least two time instants, wherein each set of at least two time instants amongst the sets of at least two time instants lie within a corresponding inter-blink time interval of the user's eyes for a corresponding blink of the user's eye, wherein the user's eye blink duration lies within a predefined range.

In this regard, measuring the temperature of the ocular surface of the user's eye at sets of at least two time instants, enables the at least one processor to accurately assess the ocular surface's temperature dynamics in relation to the natural blink cycle. By ensuring that measurements are taken within inter-blink intervals and that blinks fall within a predefined duration range, the at least one processor can reliably distinguish between temperature changes due to the tear film integrity of the user's eye being broken and the temperature changes caused by any abnormal blinking patterns or external factors. Moreover, measuring the ocular surface temperature at at sets of at least two time instants within each inter-blink interval ensures that temperature readings are taken only when the eye is open, thereby providing accurate and representative data of the ocular surface temperature. The technical effect achieved by the aforementioned approach is that errors that could arise from measurements taken during blinks or when the eye is closed, are avoided.

Optionally, the at least one processor is further configured to:

associate each set of at least two time instants amongst the sets of at least two time instants with a corresponding category, based on a blink duration of the corresponding blink of the user's eye; and
determine an average rate of change in the measured temperature, based on the rate of change in the measured temperature for each set of at least two time instants amongst the sets of pairs of time instants, wherein the rate of change is the normalized rate of change.

In this regard, by associating each set of at least two time instants with a corresponding category based on the blink duration of the corresponding blink of the user's eye, and determining an average rate of change in the measured temperature using the normalized rate of change for each set, the at least one processor can account for variations in blink patterns that affect the rate of temperature change of the ocular surface. Using the normalized rate of change reduces the impact of irregularities and anomalies in blink patterns, minimizing noise and variability in the measured data. It may be appreciated that the rate of change in the measured temperature can be normalized using any suitable normalization technique. For example, the normalized rate of change may be obtained by adjusting the measured rate of change for each set according to the baseline rate of change typical for the corresponding blink duration category. The technical effect is that normalization enables consistent and reliable comparison across different blink patterns, improving the accuracy of the assessment of the tear film integrity of the user's eye.

Optionally, the time duration is one of: 30 minutes, 1 hour, 5 hours.

Optionally, the second threshold value is determined from a predetermined average temperature of the ocular surface of the user's eye for a duration of at least 2 hours.

Optionally, the processor is further configured to adjust the measured temperature of the ocular surface of user's eye based on at least one of: a blink rate, a blink duration, an ambient temperature, an ambient lighting, a type of environment, a type of activity associated with the user's eye.

Additional aspects, advantages, features and objects of the present disclosure would be made apparent from the drawings and the detailed description of the illustrative embodiments constructed in conjunction with the appended claims.

DETAILED DESCRIPTION OF THE DRAWINGS

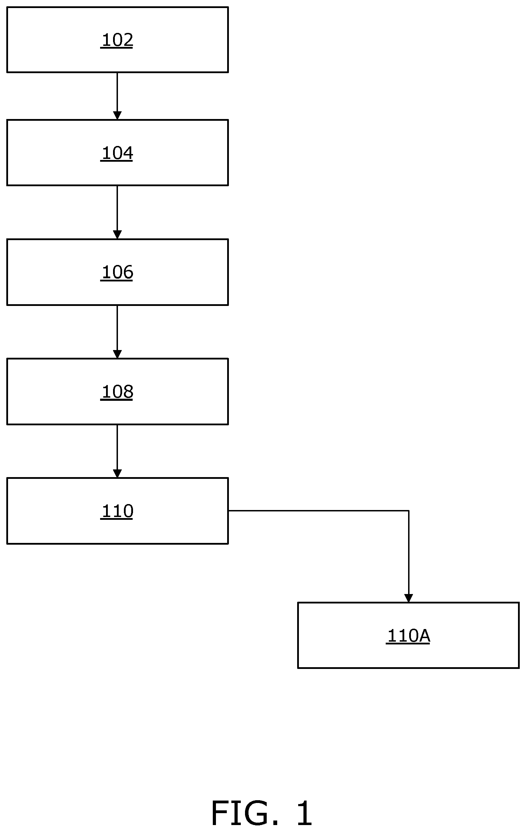

Referring to FIG. 1, illustrated is a flowchart depicting steps of a method for continual monitoring of a tear film integrity of a user's eye, in accordance with an embodiment of the present disclosure. At step **102,** a blink pattern of the user's eye is determined, using a means for detecting blinks of the user's eye. At step **104,** a temperature of an ocular surface of the user's eye is measured, at a set of at least two time instants within an inter-blink time interval of the user's eye, using at least one temperature sensor. At step **106,** a rate of change in the measured temperature for the set of at least two time instants is determined, based on a time interval between the set of at least two time instants. At step **108,** the rate of change in the measured temperature is compared with a first threshold value. At step **110,** when the rate of change in the measured temperature is greater than the first threshold value, then at step **110A,** the tear film integrity of the user's eye is deemed as broken.

The aforementioned steps are only illustrative, and other alternatives can also be provided where one or more steps are added, one or more steps are removed, or one or more steps are provided in a different sequence without departing from the scope of the claims herein.

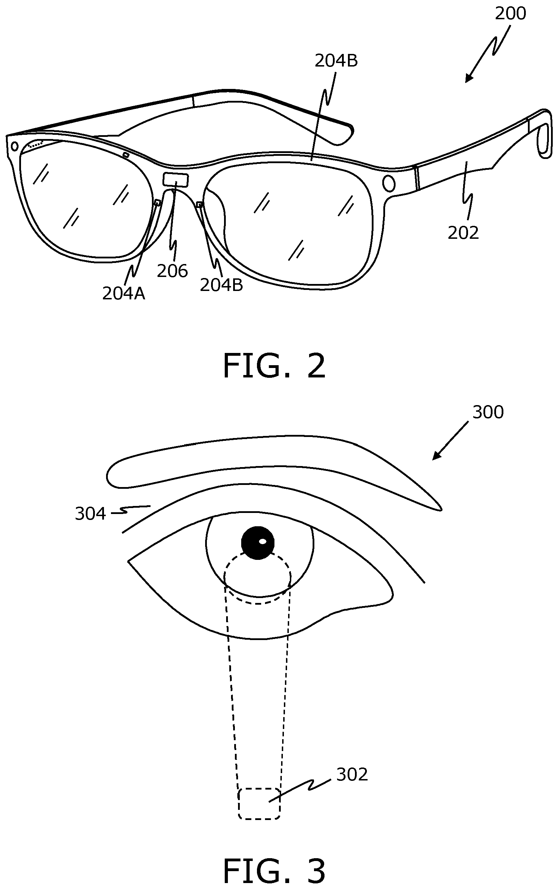

Referring to FIG. 2, illustrated is a schematic illustration of an optical apparatus **200,** in accordance with an embodiment of the present disclosure. As shown, the optical apparatus **200** comprises a frame **202.** Moreover, the optical apparatus **200** comprises at least one temperature sensor (depicted as a first temperature sensor **204A** and a second temperature sensor **204B**) arranged on the frame **202,** wherein when the optical apparatus **200** is in use, the at least one temperature sensor **204A-B** faces a user's eye. Furthermore, the optical apparatus **200** comprises at least one processor (depicted as a processor **206**) coupled to the at least one temperature sensor **204A-B,** wherein the at least one processor **206** is configured to determine a blink pattern of the user's eye, using a means for detecting blinks of the user's eye. Moreover, the at least one processor **206** is configured to control the at least one temperature sensor **204A-B** to measure a temperature of an ocular surface of the user's eye, at a set of at least two time instants within an inter-blink time interval of the user's eye. Furthermore, the at least one processor **206** is configured to determine a rate of change in the measured temperature for the set of at least two time instants, based on a time interval between the set of at least two time instants. Furthermore, the at least one processor **206** is configured to compare the rate of change in the measured temperature with a first threshold value. Furthermore, when the rate of change in the measured temperature is greater than the first threshold value the at least one processor **206** is configured to deem the tear film integrity of the user's eye as broken.

FIG. 2 is merely an example, which should not unduly limit the scope of the claims herein. It is to be understood that the specific implementations of the optical apparatus **200** are provided as examples and are not to be construed as limiting it to specific numbers, sizes, or shapes of the at least one temperature sensor **204A** and **204B,** the at least one processor **206,** and similar. A person skilled in the art will recognize many variations, alternatives, and modifications of embodiments of the present disclosure.

Referring to FIG. 3, illustrated is a schematic illustration of a user's eye **300** when an optical apparatus is in use, in accordance with an embodiment of the present disclosure. As shown, the temperature of an ocular surface of the user's eye **300** is measured, using at least one temperature sensor (depicted as a first temperature sensor **302**).

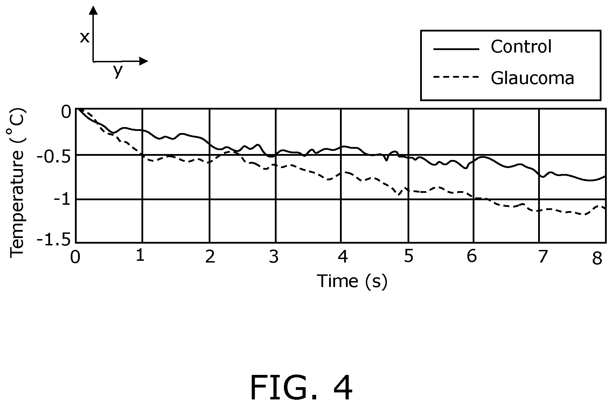

Referring to FIG. 4, illustrated is a graphical representation of a first rate of change (depicted as a solid line) and a second rate of change (depicted as a dotted line) in a measured temperature of an ocular surface of a user's eye, in accordance with an embodiment of the present disclosure. As shown, x-axis depicts an inter-blink time interval of 8 seconds, and y-axis depicts a change in the measured temperature.

## Claims

1. A method for continual monitoring of a tear film integrity of a user's eye (300), the method comprising:

   determining a blink pattern of the user's eye, using a means for detecting blinks of the user's eye;
   measuring a temperature of an ocular surface of the user's eye, at a set of at least two time instants within an inter-blink time interval of the user's eye, using at least one temperature sensor (204A, 204B, 302);
   determining a rate of change in the measured temperature for the set of at least two time instants, based on a time interval between the set of at least two time instants;
   comparing the rate of change in the measured temperature with a first threshold value; and
   when the rate of change in the measured temperature is greater than the first threshold value:
   deeming the tear film integrity of the user's eye as broken.

2. A method of claim 1, wherein the time interval between the set of at least two time instants is of at least 10 seconds.

3. A method of claim 1 or 2, wherein the first threshold value is of 0.04° Celsius per second (C/s).

4. A method of any of the preceding claims, further comprising:

   measuring the temperature of the ocular surface of the user's eye (300) at sets of at least two time instants, wherein each set of at least two time instants amongst the sets of at least two time instants lie within a corresponding inter-blink time interval of the user's eyes for a corresponding blink of the user's eye;
   determining the rate of change in the measured temperature for each set of at least two time instants amongst the sets of at least two time instants;
   determining an average rate of change in the measured temperature, based on the rate of change in the measured temperature for each set of at least two time instants amongst the sets of at least time instants;

comparing the average rate of change in the measured temperature with the first threshold value; and
when the average rate of change in the measured temperature is greater than the first threshold value:
deeming the tear film integrity of the user's eye as broken.

5. A method of any of the claims 1-3, further comprising:

measuring the temperature of the ocular surface of the user's eye (300) at sets of at least two time instants, wherein each set of at least two time instants amongst the sets of at least two time instants lie within a corresponding inter-blink time interval of the user's eyes for a corresponding blink of the user's eye whose blink duration lies within a predefined range;
determining the rate of change in the measured temperature for each set of at least two time instants amongst the sets of at least two time instants;
determining an average rate of change in the measured temperature, based on the rate of change in the measured temperature for each set of at least two time instants amongst the sets of at least time instants;
comparing the average rate of change in the measured temperature with the first threshold value; and
when the average rate of change in the measured temperature is greater than the first threshold value:
deeming the tear film integrity of the user's eye as broken.

6. A method of any of the claims 1-3, further comprising:

measuring the temperature of the ocular surface of the user's eye (300) at sets of at least two time instants, wherein each set of at least two time instants amongst the sets of at least two time instants lie within a corresponding inter-blink time interval of the user's eyes for a corresponding blink of the user's eye;
associating each set of at least two time instants amongst the sets of at least two time instants with a corresponding category, based on a blink duration of the corresponding blink of the user's eye;
determining the rate of change in the measured temperature for each set of at least two time instants amongst the sets of at least two time instants;
normalizing the determined rate of change of the measured temperature for each set of least two time instants, based on the determined corresponding category associated with said set of at least two time instants;
determining an average rate of change in the measured temperature, based on the normalized rate of change in the measured temperature for each set of at least two time instants amongst the sets of at least time instants;
comparing the average rate of change in the measured temperature with the first threshold value; and
when the average rate of change in the measured temperature is greater than the first threshold value:
deeming the tear film integrity of the user's eye as broken.

7. A method for continuous monitoring of a tear film integrity of a user's eye (300), the method comprising:

determining a blink pattern of the user's eye, using a means for detecting blinks of the user's eye;
measuring a temperature of an ocular surface of the user's eye, at a plurality of time instants during a time duration, wherein said time instant lies within an inter-blink time interval of the user's eye, using at least one temperature sensor (204A, 204B, 302);
determining an average temperature of the ocular surface of the user's eye for the time duration, based on the measured temperature of the ocular surface at the plurality of time instants;
comparing the average temperature of the ocular surface of the user's eye for the time duration with a second threshold value; and
when the average temperature of the ocular surface of the user's eye for the time duration is greater than the second threshold value:
deeming the tear film integrity of the user's eye as broken.

8. A method of claim 7, wherein the time duration is one of: 30 minutes, 1 hour, 5 hours.

9. A method of claim 7 or 8, wherein the second threshold value is determined from a predetermined average temperature of the ocular surface of the user's eye (300) for a duration of at least 2 hours.

10. A method of any of the claims 7-10, further comprising adjusting the measured temperature of the ocular surface of user's eye (300) based on at least one of: a blink rate, a blink duration, an ambient temperature, an ambient lighting, a type of environment, a type of activity associated with the user's eye.

**11.** An optical apparatus (200) comprising:

a frame (202);
at least one temperature sensor (204A, 204B, 302) arranged on the frame, wherein when the optical apparatus is in use, the at least one temperature sensor faces a user's eye (300); and
at least one processor (206) coupled to the at least one temperature sensor, wherein the at least one processor is configured to:

determine a blink pattern of the user's eye, using a means for detecting blinks of the user's eye;
control the at least one temperature sensor to measure a temperature of an ocular surface of the user's eye, at a set of at least two time instants within an inter-blink time interval of the user's eye, wherein the set of at least two time instants is sets of at least two time instants and each set of at least two time instants amongst the sets of at least two time instants lie within a corresponding inter-blink time interval of the user's eyes for a corresponding blink of the user's eye;
determine a rate of change in the measured temperature for the set of at least two time instants, based on a time interval between the set of at least two time instants, wherein the set of at least two time instants is each set of at least two time instants amongst the sets of at least two time instants;
determine an average rate of change in the measured temperature, based on the rate of change in the measured temperature for each set of at least two time instants amongst the sets of at least time instants;
compare the rate of change in the measured temperature with a first threshold value, wherein the rate of change is the average rate of change; and
when the rate of change in the measured temperature is greater than the first threshold value, wherein the rate of change is the average rate of change:
deem the tear film integrity of the user's eye as broken.

**12.** An optical apparatus (200) of claim 11, wherein the at least one processor (206) is further configured to:

measure the temperature of the ocular surface of the user's eye (300) at sets of at least two time instants, wherein each set of at least two time instants amongst the sets of at least two time instants lie within a corresponding inter-blink time interval of the user's eyes for a corresponding blink of the user's eye, wherein the user's eye blink duration lies within a predefined range

**13.** An optical apparatus (200) of claim 11 or 12, wherein the at least one processor (206) is further configured to:

associate each set of at least two time instants amongst the sets of at least two time instants with a corresponding category, based on a blink duration of the corresponding blink of the user's eye; and
determine an average rate of change in the measured temperature, based on the rate of change in the measured temperature for each set of at least two time instants amongst the sets of pairs of time instants, wherein the rate of change is the normalized rate of change.

**14.** An optical apparatus (200) comprising:

a frame (202);
at least one temperature sensor (204A, 204B, 302) arranged on the frame, wherein when the optical apparatus is in use, the at least one temperature sensor faces a user's eye (300); and
at least one processor (206) coupled to the at least one temperature sensor, wherein the at least one processor is configured to:

determine a blink pattern of the user's eye, using a means for detecting blinks of the user's eye;
control the at least one temperature sensor to measure a temperature of an ocular surface of the user's eye, at a plurality of time instants during a time duration, wherein said time instant lies within an inter-blink time interval of the user's eye, and wherein the measured temperature of the ocular surface of user's eye (300) is adjusted based on at least one of: a blink rate, a blink duration, an ambient temperature, an ambient lighting, a type of environment, a type of activity associated with the user's eye;
determine an average temperature of the ocular surface of the user's eye for the time duration, based on the measured temperature of the ocular surface at the plurality of time instants;
compare the average temperature of the ocular surface of the user's eye for the time duration with a second threshold value; and

when the average temperature of the ocular surface of the user's eye for the time duration is greater than the second threshold value:
deem the tear film integrity of the user's eye as broken.

```
┌─────────────────────────┐
│                         │
│          102            │
│                         │
└─────────────────────────┘
             │
             ▼
┌─────────────────────────┐
│                         │
│          104            │
│                         │
└─────────────────────────┘
             │
             ▼
┌─────────────────────────┐
│                         │
│          106            │
│                         │
└─────────────────────────┘
             │
             ▼
┌─────────────────────────┐
│                         │
│          108            │
│                         │
└─────────────────────────┘
             │
             ▼
┌─────────────────────────┐
│                         │
│          110            ├──────────┐
│                         │          │
└─────────────────────────┘          │
                                      ▼
                          ┌─────────────────────────┐
                          │                         │
                          │         110A            │
                          │                         │
                          └─────────────────────────┘
```

# FIG. 1

FIG. 2

FIG. 3

FIG. 4